# EUROPÄISCHE PATENTANMELDUNG

(11) **EP 4 443 132 A1**
(43) Veröffentlichungstag der Anmeldung: **09.10.2024**
(21) Anmeldenummer: 23167073.8
(22) Anmeldetag: 06.04.2023
(51) Int. Cl.: G01N 11/00, G01N 3/00, G01N 33/10

(54) **TEIGPROBENMESSSYSTEM MIT EINEM TRANSLATORISCH BEWEGBAREN TEIGSCHALENTRÄGER**

(71) Anmelder: Brabender GmbH & Co. KG, 47055 Duisburg (DE)
(72) Erfinder: Berresheim, Bernd, 46459 Rees (DE); Brandt, Marcel, 47443 Moers (DE); Bulert, Tobias, 42111 Wuppertal (DE)
(74) Vertreter: Michalski Hüttermann & Partner Patentanwälte mbB

(57) **Zusammenfassung**

Die vorliegende Erfindung betrifft ein Teigprobenmesssystem zur Bestimmung von Eigenschaften einer Teigprobe, wobei das Teigprobenmesssystem (10) mindestens aufweist:
- eine Hubeinrichtung (12), welche entlang einer Hubachse (H) derart in unterschiedliche Hubpositionen translatorisch höhenverstellbar ist, dass die Teigprobe mit einer Translationskraft beaufschlagbar ist;
- ein ortsfestes Kraftbeaufschlagungsmittel (14), um die Teigprobe entlang einer Kraftbeaufschlagungshauptachse (K) infolge der translatorischen Bewegung der Teigaufnahmeeinrichtung (16) mit der Translationskraft zu beaufschlagen;
- eine entlang einer Teigaufnahmenlängsachse (T) translatorisch bewegbare Teigaufnahmeeinrichtung (16), um die Teigprobe tragend widerzulagern,
wobei die Teigaufnahmeeinrichtung (16) derart mit der Hubeinrichtung (12) verbunden ist, dass die Teigaufnahmeeinrichtung (16) bei einer Änderung der Hubposition der Hubeinrichtung (12) entlang der Hubachse (H) ihrerseits entlang der Teigaufnahmenlängsachse (T) translatorisch bewegbar ausgebildet ist;
- ein Lastsensorsystem (18) zur Detektion der Translationskraft entlang einer Lastdetektionshauptachse (L), wobei das Lastsensorsystem (18) zumindest mittelbar mit dem Kraftbeaufschlagungsmittel (14) verbunden ist.

## Beschreibung

### Technisches Gebiet

Die vorliegende Erfindung betrifft ein Teigprobenmesssystem zur Bestimmung von Eigenschaften einer Teigprobe.

Des Weiteren betrifft die vorliegende Erfindung ein Verfahren zur Bestimmung von Eigenschaften einer Teigprobe mit dem vorgenannten Teigprobenmesssystem.

Des Weiteren betrifft die vorliegende Erfindung ein Computerprogramm, ein Datenträgersignal und ein computerlesbares Medium.

### Hintergrund der Erfindung

Bislang bekannt sind Teigprobenmesssysteme und Verfahren zur Bestimmung der rheologischen Eigenschaften von Teigproben, beispielsweise Weizenmehlteigproben, im Rahmen von Dehnversuchen. Eine dabei aufgenommene Belastungs-Dehnungskurve wird zur Bestimmung der allgemeinen Qualität eines Mehles und seiner Reaktion auf den Zusatz von Backmitteln verwendet. Diese Methoden sind für Mehl, bevorzugt aus Weizen, beispielsweise Triticum aestivum L., anwendbar.

Hierzu ist vorgesehen, dass ein Teigprobenmesssystem ein translatorisch bewegbares Kraftbeaufschlagungsmittel und eine ortsfeste Teigaufnahmeeinrichtung zur Widerlagerung der Teigprobe aufweist. Grundsätzlich sind das Kraftbeaufschlagungsmittel und die Teigaufnahmeeinrichtung derart ausgebildet, dass sich das Kraftbeaufschlagungsmittel und die Teigaufnahmeeinrichtung translatorisch vertikal annähern und insbesondere durch eine entsprechende geometrische Ausgestaltung der beiden Komponenten auch durchfahren beziehungsweise, dass sich das Kraftbeaufschlagungsmittel während seiner weiteren Bewegung von der Teigaufnahmeeinrichtung entfernt.

Sowohl die Teigaufnahmeeinrichtung als auch die darauf positionierte Teigprobe sind über einen Hebelarm vom Lastsensorsystem entfernt. Wie aus der Messtechnik bekannt ist, wächst das Maß von Messungenauigkeiten mit einer zunehmenden Zahl von Faktoren, die in den Messprozess einwirken. Der Einfluss des Hebelarms verstärkt die Messungenauigkeiten dabei signifikant. Da unter anderem diese Fehlerquelle als Faktor aus Kraft und Hebelarm ein bislang nicht lösbares Problem darstellt, wird das Teigprobenmesssystem vor jedem Messdurchlauf tariert. Zudem muss die als Teigschale ausgebildete Teigaufnahmeeinrichtung mitsamt der Teigprobe vor jedem Messprozess fixiert werden, da der Hebelarm Messungenauigkeiten deutlich verstärkt. Als weiterer wechselhafter Einflussfaktor zeigt sich das variierende Reißverhalten durch eine Vertikalbewegung der Teigprobe in der Teigaufnahmeeinrichtung.

### Beschreibung der Erfindung

Ausgehend von dieser Situation ist es eine Aufgabe der vorliegenden Erfindung, ein verbessertes Teigprobenmesssystem zu schaffen, welches insbesondere eine schneller durchführbare und somit günstigere Teigprobenmessung bei höherer Messqualität ermöglicht.

Die vorgenannte Aufgabe der Erfindung wird durch die Merkmale der unabhängigen Hauptansprüche gelöst. Vorteilhafte Ausgestaltungen sind in den Unteransprüchen angegeben. Sofern technisch möglich, können die Lehren der Unteransprüche beliebig mit den Lehren der Haupt- und Unteransprüche kombiniert werden.

Insbesondere wird die Aufgabe demnach gelöst durch ein Teigprobenmesssystem zur Bestimmung von Eigenschaften einer Teigprobe. Das Teigprobenmesssystem weist hierzu mindestens auf:
- eine Hubeinrichtung, welche entlang einer Hubachse derart in unterschiedliche Hubpositionen translatorisch höhenverstellbar ist, dass die Teigprobe abhängig von der Hubposition der Hubeinrichtung infolge einer translatorischen Relativbewegung zwischen einem ortsfesten Kraftbeaufschlagungsmittel und einer translatorisch bewegbaren Teigaufnahmeeinrichtung mit einer Translationskraft beaufschlagbar ist;
- ein ortsfestes Kraftbeaufschlagungsmittel, um die Teigprobe entlang einer Kraftbeaufschlagungshauptachse infolge der translatorischen Bewegung der Teigaufnahmeeinrichtung widerlagernd mit der Translationskraft zu beaufschlagen;
- eine entlang einer Teigaufnahmenlängsachse translatorisch bewegbare Teigaufnahmeeinrichtung, um die Teigprobe tragend widerzulagern,
   wobei die Teigaufnahmeeinrichtung derart mit der Hubeinrichtung verbunden ist, dass die Teigaufnahmeeinrichtung bei einer Änderung der Hubposition der Hubeinrichtung entlang der Hubachse ihrerseits entlang der Teigaufnahmenlängsachse translatorisch bewegbar ausgebildet ist;
- ein Lastsensorsystem zur Detektion der Translationskraft entlang einer Lastdetektionshauptachse, wobei das Lastsensorsystem zumindest mittelbar mit dem Kraftbeaufschlagungsmittel verbunden ist.

Nachfolgend werden vorteilige Aspekte des Teigprobenmesssystems erläutert und weiter nachfolgend bevorzugte modifizierte Ausführungsformen des Teigprobenmesssystems beschrieben. Erläuterungen, insbesondere zu Vorteilen und Definitionen von Merkmalen, sind dem Grunde nach beschreibende und bevorzugte, jedoch nicht limitierende Beispiele. Sofern eine Erläuterung limitierend ist, wird dies ausdrücklich erwähnt.

Da zum Beispiel die Teigprobe, welche Gegenstand des Messvorgangs ist, mit der Teigaufnahmeeinrichtung selbst hoch- oder runterbewegt werden kann, was mit Vorzug durch den Einfluss der Teigprobengewichtskraft selbststabilisierend wirkt, reduziert sich das Risiko von ungewollten Querkräften und/oder Momenten, welche auf eine stillstehende Teigprobe einwirken.

Mit Vorteil reduziert sich bei spröden Teigproben, etwa bei Keksen, deren Kippeligkeit, sodass auch in diesem Kontext die Messqualität erhöht wird.

Es zeigt sich insgesamt, dass der Einfluss von horizontalen Kräften reduziert wird, wenn sich die Teigprobe selbst vertikal bewegt und hierdurch, insbesondere auch unter dem fixierenden Einfluss deren Gewichtskraft, in ihrer Lage stabilisiert wird.

Das Teigprobenmesssystem dient insbesondere zur Bestimmung von rheologischen Eigenschaften einer Teigprobe. Die zu bestimmenden rheologischen Eigenschaften der Teigproben umfassen insbesondere deren Verformungs- und Fließverhalten. Die Rheologie betrifft insbesondere Teilgebiete der Elastizitätstheorie, der Plastizitätstheorie und der Strömungsmechanik.

Insbesondere wird das Teigprobenmesssystem bei harten Teigproben, beispielsweise Keksen, für Druckversuche verwendet. Bei weichen Teigproben, beispielsweise Mehl-Wasser-Mischung, wird das Teigprobenmesssystem insbesondere für Zugversuche verwendet. Dabei kann es sein, dass das Teigprobenmesssystem nur für eine der beiden Versuchsarten genutzt wird oder auch für beide Versuchsarten nutzbar ist. Hierzu sind bevorzugt das Kraftbeaufschlagungsmittel und/oder die Teigaufnahmeeinrichtung austauschbar, also modular, ausgebildet. Ein entsprechendes Rechensystem steuert die entsprechenden Versuche.

Als Teigprobe kann ein Mehlteig dienen, beispielsweise ein Weizenmehlteig, insbesondere aufweisend Triticum aestivum L., welcher eine Mehl-Wasser-Mischung aufweist. Dieser Mehlteig kann durch die Bewegung zwischen dem Kraftbeaufschlagungsmittel und der Teigaufnahmeeinrichtung auf Zug beansprucht werden und sich bis zum Reißen dehnen.

Ein Vorteil des vorliegenden Teigprobenmesssystems liegt in seiner vielseitigen Anwendbarkeit. So kann als Teigprobe auch ein ausgebackener Keks verwendet werden, welcher anstelle der konventionellen Zugmessung durch die Bewegung zwischen dem Kraftbeaufschlagungsmittel und der Teigaufnahmeeinrichtung einer Druckmessung unterzogen werden kann. In dem Fall ist insbesondere vorgesehen, dass die bewegbare Teigaufnahmeeinrichtung das ortsfeste Kraftbeaufschlagungsmittel nicht durchfährt. Das Teigprobenmesssystem umfasst zur Teigprobenmessung mindestens die Hubeinrichtung, das Kraftbeaufschlagungsmittel, die Teigaufnahmeeinrichtung und das Lastsensorsystem.

Die Hubeinrichtung ist entlang ihrer Hubachse in unterschiedliche Hubpositionen translatorisch höhenverstellbar. Da die Teigprobe auf der Teigaufnahmeeinrichtung liegt, welche mit der Hubeinrichtung verbunden ist, ergibt sich die translatorische Bewegung der Teigaufnahmeeinrichtung und somit der Teigprobe aus der translatorischen Bewegung der Hubeinrichtung. Dies kann unmittelbar oder mittelbar erfolgen. Beispielhaft kann ein Verbindungsmittel oder eine Getriebeanordnung zwischen der Teigaufnahmeeinrichtung und der Hubeinrichtung angeordnet sein. Die auf die Teigprobe wirkende Translationskraft resultiert nach alledem hauptsächlich aus der Hubbewegung der Hubeinrichtung, welche über die Hubbewegung der bewegbaren Teigaufnahmeeinrichtung entgegen dem ortsfesten Kraftbeaufschlagungsmittel wirkt. Die Teigaufnahmeeinrichtung und das Kraftbeaufschlagungsmittel sind derart korrelierend ausgebildet, dass sie bei einer vertikal durchgehenden Bewegung der Teigaufnahmeeinrichtung durch das ortsfeste Kraftbeaufschlagungsmittel nicht kollidieren, sodass der etwaige Kraftfluss zwischen der Teigaufnahmeeinrichtung und dem Kraftbeaufschlagungsmittel über die dazwischen angeordnete Teigprobe erfolgt. Das Kraftbeaufschlagungsmittel überträgt diese Kraft zur Lastdetektion in das Lastsensorsystem. Mit anderen Worten ist die Teigprobe abhängig von der Hubposition der Hubeinrichtung infolge der translatorischen Relativbewegung zwischen dem ortsfesten Kraftbeaufschlagungsmittel und der translatorisch bewegbaren Teigaufnahmeeinrichtung mit der zu detektierenden Translationskraft beaufschlagbar.

Als Relativbewegung zwischen dem ortsfesten Kraftbeaufschlagungsmittel und der translatorisch bewegbaren Teigaufnahmeeinrichtung ist mit anderen Worten zu verstehen, dass sich die Teigaufnahmeeinrichtung vertikal in Richtung des ortsfesten Kraftbeaufschlagungsmittels bewegt. Dabei verbleibt die Teigprobe, soweit möglich, unbewegt auf der Teigaufnahmeeinrichtung. Erst wenn die Teigprobe auf das ortsfeste Kraftbeaufschlagungsmittel trifft, ist grundsätzlich eine Verformung der Teigprobe vorgesehen. Es kann unter bestimmten Messumständen gewünscht oder zumindest legitim sein, dass die Teigprobe schon vor dem Kontakt verformt. Es ist doch auch möglich, dass dieser Messumstand explizit auszuschließen ist.

Um die Vielseitigkeit des Teigprobenmesssystems zu erhöhen, eignet sich mit Vorteil ein maximaler Hub der Hubeinrichtung von im Wesentlichen 80 Zentimetern. Im Wesentlichen betrifft hierbei einen Spielraum von zehn Prozent nach oben oder unten, was bevorzugt einem mindesten Maximalhub von 72 Zentimetern und einem maximalen Maximalhub von 88 Zentimetern entspricht. Ein geringerer Hub als 72 Zentimeter hat sich für die Vielseitigkeit als weniger vorteilhaft herausgestellt und ein höherer Hub als 88 Zentimeter führt zu unwirtschaftlichen Mehrkosten des Teigprobenmesssystems ohne nennenswerten Mehrwert. Insbesondere kann der maximale Hub der Hubeinrichtung genau 80 Zentimeter betragen.

Bevorzugt ist die Hubeinrichtung teleskopierbar ausgestaltet, um eine kompakte Bauweise des Teigprobenmesssystems zu ermöglichen.

Als Translationskraft gilt insbesondere jene Kraft, welche vom ortsfesten Kraftbeaufschlagungsmittel auf die jeweilige Teigprobe wirkt. Strukturell ist das Kraftbeaufschlagungsmittel ortsfest, doch liegt die Teigprobe auf der bewegbaren Teigaufnahmeeinrichtung auf und kann entlang der Teigaufnahmenlängsachse hochbeziehungsweise heruntergefahren werden und sich dem Kraftbeaufschlagungsmittel damit nähern oder sich von diesem entfernen. Wenn nun die Teigaufnahmeeinrichtung an das Kraftbeaufschlagungsmittel heranbewegt wird, wirkt ab einem Moment des Kontakts zwischen dem Kraftbeaufschlagungsmittel und der Teigprobe von der sich bewegenden Teigprobe eine Kraft auf das ortsfeste Kraftbeaufschlagungsmittel. Umgekehrt wirkt das ortsfeste Kraftbeaufschlagungsmittel mit einer wechselwirkenden Kraft auf die Teigprobe. Das fachmännisch bekannte dritte Newtonsche Axiom, auch bekannt als Wechselwirkungsprinzip, sieht vor, dass sich beide Kräfte im Betrag entsprechen und erlaubt daher die vorliegende Art der Verwendung der Formulierung einer auf die Teigprobe wirkenden Translationskraft, sofern die Teigprobe mit dem ortsfesten Kraftbeaufschlagungsmittel wechselwirkt. Vorliegend wird daher vereinfacht die Formulierung Translationskraft verwendet. Es hat sich regelmäßig herausgestellt, dass es keine weiteren Einflussfaktoren gibt, welche sich gegebenenfalls störend auf das Messergebnis auswirken.

Insbesondere ist vorgesehen, dass die Teigprobe vor dem Befestigen in der Teigaufnahmeeinrichtung im Rahmen eines definierten Prozesses gefertigt wurde. Dies stellt beispielhaft sicher, dass unterschiedliche Messergebnisse nicht aus ungewollt unterschiedlichen Wasseranteilen zwischen verschiedenen Teigproben resultieren. Auch kann somit beispielhaft sichergestellt werden, dass die Teigproben stets das gleiche Gewicht, Gewichtsverteilung und/oder Form haben, sodass die Gewichtskraft und/oder andere Faktoren mit gleichem Einfluss in alle Ergebnisse einwirkt beziehungsweise einwirken. Dies sind nur Beispiele, welche darlegen, dass es sinnvoll ist, die Teigprobe zuvor unter fest definierten Bedingungen herzustellen. Selbstredend können unterschiedliche Teigproben jeweils voneinander unterschiedliche Komponentenanteile, beispielhaft Wasser- und/oder Mehlanteile, aufweisen, wenn dies dem Wesen der Messreihe entspricht. Letzteres gilt auch für andere Teigprobenparameter.

Das ortsfeste Kraftbeaufschlagungsmittel ist vorgesehen, um die Teigprobe entlang der Kraftbeaufschlagungshauptachse infolge der translatorischen Bewegung der Teigaufnahmeeinrichtung mit der Translationskraft zu beaufschlagen. Insbesondere kann als Translationskraft in diesem Sinne die gegenwirkende Kraft verstanden werden zu der Kraft, mit der die bewegte Teigprobe gegen das ortsfeste Kraftbeaufschlagungsmittel wirkt. Beispielhaft kann das ortsfeste Kraftbeaufschlagungsmittel als hakenähnliche oder hakengleiche Einrichtung oder als Plattenkonstruktion mit Ausnehmungen zum Durchlass der Teigaufnahmeeinrichtung und/oder zur gleichzeitigen kraftübertragenden Widerlagerung der Teigprobe ausgebildet sein. Grundsätzlich sind auch andere Ausgestaltungen des Kraftbeaufschlagungsmittels möglich. Somit wird die Teigprobe mit der Translationskraft beaufschlagt, indem das stillstehende Kraftbeaufschlagungsmittel der sich bewegenden Teigprobe entgegensteht.

Weiterhin umfasst das Teigprobenmesssystem eine entlang einer Teigaufnahmenlängsachse translatorisch bewegbare Teigaufnahmeeinrichtung, um die Teigprobe tragend widerzulagern. Beispielhaft kann die Teigaufnahmeeinrichtung als Plattenkonstruktion mit Ausnehmungen zur Durchfahrt der Teigaufnahmeeinrichtung und/oder zur gleichzeitigen tragenden Widerlagerung der Teigprobe ausgebildet sein. Bevorzugt ist die Teigaufnahmeeinrichtung derart tragend ausgestaltet, dass sich die Teigprobe während eines Messvorgangs nicht durch ihr Eigengewicht verformt, sondern dass durch die Gewichtskraft während der Bewegung der Teigaufnahmeeinrichtung ungewollte Bewegungen der Teigprobe, beispielhaft seitliche Fließverformungen, vermieden werden und hierdurch die Messqualität erhöht wird.

Es hat sich herausgestellt, dass robust wiederholbare Messergebnisse erzielt werden, wenn die Teigaufnahmeeinrichtung mit einer Geschwindigkeit von 14,5 mm/s plus/minus 0,5 mm/s bewegt wird.

Mit Vorteil kehrt die Teigaufnahmeeinrichtung nach Abschluss der Messung automatisch in ihre ursprüngliche Position zurück. Optional kann vorgesehen sein, dass der Antriebsmotor zur Bewegung der Teigaufnahmeeinrichtung nach dem Reißen der Teigprobe von Hand abzuschalten ist. Grundsätzlich kann der Antriebsmotor manuell oder automatisch auf Rücklauf geschaltet werden, um die Teigaufnahmeeinrichtung in die ursprüngliche Position zurückzubringen.

Bevorzugt kann die Teigprobe in der Teigaufnahmeeinrichtung eingespannt oder anderweitig befestigt werden, beispielhaft auch fixierend durch- oder angestochen. Zusätzlich oder alternativ ist es auch möglich, dass die Teigaufnahmeeinrichtung derart konkav ausgeformt ist, dass sie die Teigprobe vor und/oder während der Messung derart bettet, dass die Teigprobe horizontal nicht von der Teigaufnahmeeinrichtung zu fallen droht. Abhängig von der Art der Messung und der Teigprobe, kann die konkave Ausgestaltung unterschiedlich ausgestaltet sein.

Grundsätzlich ist es möglich, dass sich die Teigaufnahmeeinrichtung dem Kraftbeaufschlagungsmittel von oben oder von unten nähert und gegebenenfalls durchfährt. Ein Durchfahren erfordert, dass sich die die Komponenten der Teigaufnahmeeinrichtung und des Kraftbeaufschlagungsmittels beim vertikalen Durchfahren nicht schneiden, sodass die Teigaufnahmeeinrichtung und das Kraftbeaufschlagungsmittel entsprechend ausgestaltbar beziehungsweise ausgestaltet sind. Hierzu können die Teigaufnahmeeinrichtung und/oder das Kraftbeaufschlagungsmittel eine oder mehrere Ausnehmungen aufweisen.

Wenn die Teigprobe beispielhaft ein Keks ist und ausschließlich eine Druckuntersuchung vorgesehen ist, sodass das Kraftbeaufschlagungsmittel nicht von der Teigaufnahmeeinrichtung durchfahren wird, sind keine Ausnehmungen an dem Kraftbeaufschlagungsmittel und der Teigaufnahmeeinrichtung nötig. Zum Beispiel kann die Teigaufnahmeeinrichtung eine Platte sein, insbesondere mit einem konkaven Aufnahmebereich für den Keks, und das ortsfeste Kraftbeaufschlagungsmittel kann beispielhaft stößelähnlich ausgebildet sein. Sofern keine Ausnehmung vorgesehen ist, endet die Messung spätestens, wenn das Kraftbeaufschlagungsmittel und die Teigaufnahmeeinrichtung aufeinander treffen. Die Platte kann jedoch auch eine Ausnehmung aufweisen für das stößelähnliche Kraftbeaufschlagungsmittel, sodass sich das ortsfeste Kraftbeaufschlagungsmittel durch die sich bewegende Teigaufnahmeeinrichtung erstrecken kann. Dies kann sinnvoll sein, wenn Druckversuche an sehr dehnbaren Teigproben durchgeführt werden.

Die Teigaufnahmeeinrichtung ist insbesondere derart mit der Hubeinrichtung verbunden, dass die Teigaufnahmeeinrichtung bei einer Änderung der Hubposition der Hubeinrichtung entlang der Hubachse ihrerseits entlang der Teigaufnahmenlängsachse translatorisch bewegt wird.

Das Lastsensorsystem dient zur Detektion der Translationskraft entlang einer Lastdetektionshauptachse. Besonders bevorzugt entsprechen sich die Teigaufnahmenlängsachse und die Lastdetektionshauptachse und verlaufen somit koaxial. Sofern die Teigaufnahmenlängsachse und die Lastdetektionshauptachse nicht koaxial verlaufen, sind sie bevorzugt zumindest parallel zueinander ausgebildet. Wenn beispielhaft eine Teigprobe auf der bewegbaren Teigaufnahmeeinrichtung aufliegt und das ortsfeste Kraftbeaufschlagungsmittel als darüberliegender Haken ausgebildet ist, zieht die sich dehnende Teigprobe am Kraftbeaufschlagungsmittel. In einer direkten Folge zieht die Teigprobe beziehungsweise das Kraftbeaufschlagungsmittel am Lastsensorsystem, sodass die Translationskraft als Zugkraft ermittelt wird, bis die Bewegung der Teigaufnahmeeinrichtung endet oder bis die Teigprobe reißt.

Das Lastsensorsystem ist zumindest mittelbar, vorzugsweise unmittelbar, mit dem Kraftbeaufschlagungsmittel verbunden. Eine unmittelbare Verbindung hat den Vorteil, dass möglichst wenig Messeinflüsse das Messergebnis verfälschen.

Das Lastsensorsystem kann als Kraftaufnehmer ausgebildet sein, zum Beispiel als Kraftmessdose, Kraftmessring, Miniatur- oder S-förmiger Sensor. Möglich sind auch andere, gleichwirkende Kraftaufnehmer. Es ist auch möglich, unterschiedliche Kraftaufnehmer zu kombinieren, um die Qualität der Messdaten zu erhöhen, indem beispielhaft Ausreißer besser identifiziert, beseitigt und/oder ausgeglichen werden können.

Gemäß einem ersten Beispiel ist eine unten befindliche Teigschale als translatorisch bewegbare Teigaufnahmeeinrichtung mit einem längs gewirkten Mehlteigling als Teigprobe bestückt. Bei Start einer Messung verfährt die Hubeinrichtung konstant vertikal nach oben in Richtung des oben befindlichen Kraftbeaufschlagungsmittels, wodurch die Teigaufnahmeeinrichtung in Richtung des Kraftbeaufschlagungsmittels mitbewegt wird. Ab einer bestimmten Hubposition kommt die Teigprobe in Kontakt mit dem als ortsfesten Kraftbeaufschlagungsmittel ausgebildeten Zughaken. Der Zughaken ist direkt gekoppelt mit dem Lastsensorsystem, sodass der Mehlteigling über den Zughaken mit einer Translationskraft am Lastsensorsystem zieht. Abhängig vom Verfahrweg stellt sich dabei eine Dehnung im Mehlteigling ein. Die vom Lastsensorsystem gemessene Translationskraft wird konstant erfasst und über den Verfahrweg in einem Diagramm aufgetragen bis der Mehlteigling reißt.

Gemäß einem zweiten Beispiel ist die Teigaufnahmeeinrichtung vor Messbeginn vertikal unter dem als Stempel ausgebildeten Kraftbeaufschlagungsmittel angeordnet. Ein Keks liegt auf der Teigaufnahmeeinrichtung. Nun fährt die Teigaufnahmeeinrichtung hoch bis der Keks auf den Stempel trifft und bricht. Das Lastsensorsystem ist direkt mit dem Stempel verbunden und sensoriert die auf das Kraftbeaufschlagungsmittel wirkende Translationskraft.

Das Teigprobenmesssystem erlaubt mit Vorzug eine direkte Kraftmessung in der einer Zugachse entsprechenden Lastdetektionshauptachse, sodass kein Hebelarm oder allenfalls ein nur geringer Hebelarm die Messdaten negativ beeinflusst. Längere Hebelarme erzeugen bislang stetig größer werden Momente und wirken sich daher negativ auf die Genauigkeit und Aussagekraft der Messergebnisse aus.

Bevorzugt ist die Teigaufnahmeeinrichtung modular aufgebaut und kann variable Formen aufweisen, um die Teigprobe tragend widerzulagern.

Bevorzugt ist das Lastsensorsystem derart fix am Teigprobenmesssystem montiert, dass auf dem Lastsensorsystem über eine modulare Schnittstelle Haken, Teller oder ähnliche Geometriemittel als Kraftbeaufschlagungsmittel lösbar befestigt werden können.

Bevorzugt ist die Teigaufnahmeeinrichtung lösbar mit dem Teigprobenmesssystem verbunden. Dies erleichtert die Montage der Teigaufnahmeeinrichtung insofern, als die Hubeinrichtung derart verfahren werden kann, dass die Teigaufnahmeeinrichtung für den Maschinenbediener gut zugänglich ist. Es ist möglich, die Teigaufnahmeeinrichtung allein zu montieren und die Teigprobe später zu fixieren. Alternativ kann die Teigaufnahmeeinrichtung zuerst mit der Teigprobe fixiert werden und anschließend gemeinsam mit dem Teigprobenmesssystem verbunden werden.

Bevorzugt ist kein konventionell verwendetes Wegmesssystem notwendig, um die Translationsstrecke zu bestimmen. Stattdessen kann die Translationsstrecke aus einer Motor- und/oder Getriebetätigkeit hergeleitet werden, beispielsweise hergeleitet aus dem erforderlichen Motorstrom und/oder den Getriebekomponentenumdrehungen.

Auch ermöglicht das Teigprobenmesssystem ein kompaktes Gerätedesign und damit geringe Verpackungsmaße.

Bevorzugt ist die Hubeinrichtung beliebig im beziehungsweise am Gerät positionierbar, sodass das Teigprobenmesssystem flexibel gestaltet werden kann. Vorteilig ist vorgesehen, dass die Hubeinrichtung und die Teigaufnahmeeinrichtung derart mittelbar oder unmittelbar miteinander verbunden sind, dass eine Änderung der Hubposition der Hubeinrichtung zu einer Änderung des Hubs der Teigaufnahmeeinrichtung und somit der Teigprobe führt.

Bevorzugt sind die Verfahrwege der Hubeinrichtung und/oder der Teigaufnahmeeinrichtung abdeckbar und/oder beliebig erweiterbar. Dies erweitert die Einsatzmöglichkeiten des Teigprobenmesssystems. So kann zum Beispiel die Hubeinrichtung zunächst nur einen Maximalhub von 72 Zentimetern aufweisen. Wird allerdings für eine bestimmte Messung ein Maximalhub von 88 Zentimetern erfordert, kann eine Verlängerungsvorrichtung von mindestens 16 Zentimetern angefügt werden. Sobald die erforderlichen Messreihen abgeschlossen sind, kann die Verlängerungsvorrichtung entfernt werden, um die kompakte Bauweise des Teigprobenmesssystems weiter sicherzustellen. Um Verletzungen oder sonstige Beschädigungen zu vermeiden, kann die Hubeinrichtung mit einer Ummantelung abgedeckt werden, welche optional ebenfalls erweiterbar ist.

Es hat sich herausgestellt, dass die Entkopplung des Lastsensorsystems vom Teigaufnahmeeinrichtung die Fehleranfälligkeit reduziert.

Vorteilhafterweise muss der Teigschalenträger nicht fixiert werden. Abhängig von der Art der Messungen, kann dies jedoch vorgesehen sein.

Sofern das Kraftbeaufschlagungsmittel als Haken ausgebildet ist, ermöglicht dies einen kurzen Hebelarm. Im Falle einer modularen Ausgestaltung des Kraftbeaufschlagungsmittels, ist dieses einfach auszutauschen und das Teigprobenmesssystem vielseitig nutzbar.

Die vielseitige Nutzbarkeit des Teigprobenmesssystems zeigt sich unter anderem darin, dass es unterschiedliche Messcharakteristiken zulässt, beispielsweise betreffend Zug- oder Druckmessungen.

Mit Vorteil ist gegenüber Stand der Technik- Teigprobenmesssystemen keine zusätzliche Tarierung notwendig.

Mit weiterem Vorteil sind Mehrfachmessungen in einem Bewegungsablauf möglich, sodass eine Erweiterung um weitere Messstellen vorliegt.

Bevorzugt gibt es modular wechselbare Zug-/ Druckmodule. Somit kann das gleiche Teigprobenmesssystem nach einer Modifikation z. B. Knusprigkeit eines Kekses vermessen und die Dehnbarkeit eines Mehl-Wasser-Gemisches bestimmen.

Das ortsfeste Kraftbeaufschlagungsmittel und die translatorisch bewegbare Teigaufnahmeeinrichtung sind derart ausgebildet, dass sich die sich bewegende Teigaufnahmeeinrichtung dem Kraftbeaufschlagungsmittel zunächst translatorisch vertikal annähert. Ab einem bestimmten Punkt blockieren sich Teigaufnahmeeinrichtung und Kraftbeaufschlagungsmittel nicht, sondern die Teigaufnahmeeinrichtung durchfährt das Kraftbeaufschlagungsmittel, da Teigaufnahmeeinrichtung und Kraftbeaufschlagungsmittel korrelierende geometrische Ausgestaltungen, insbesondere Ausnehmungen, aufweisen, die ein Durchfahren der Teigaufnahmeeinrichtung durch das ortsfeste Kraftbeaufschlagungsmittel ermöglichen. Im weiteren Bewegungsverlauf der Teigaufnahmeeinrichtung entfernt sich diese translatorisch vertikal von dem Kraftbeaufschlagungsmittel.

Üblicherweise ist vorgesehen, dass die Teigaufnahmeeinrichtung das ortsfeste Kraftbeaufschlagungsmittel für einen Messprozess einer nicht wärmebehandelten Mehlteigprobe von unten nach oben durchfährt. Hierzu weist die Teigaufnahmeeinrichtung beispielsweise seitlich zwei Auflageabschnitte auf, wobei sich die Mehlteigprobe über beide Auflageabschnitte erstreckt. Zwischen den Auflageabschnitten weist die Teigaufnahmeeinrichtung eine Materialausnehmung auf.

Analog hierzu ist das Kraftbeaufschlagungsmittel, beispielhaft als Zughaken, derart ausgebildet und positioniert, dass das Kraftbeaufschlagungsmittel bei einer translatorischen Bewegung der Teigaufnahmeeinrichtung nach oben zwischen den beiden Auflageabschnitten verbleibt, sodass Teigaufnahmeeinrichtung und Kraftbeaufschlagungsmittel nicht kollidieren. Wenn die Teigaufnahmeeinrichtung nun hochfährt, trägt sie die Mehlteigprobe. Sobald die Mehlteigprobe auf das Kraftbeaufschlagungsmittel trifft, beispielsweise der Zughaken, wird sie entsprechend gedehnt. Hierdurch wirkt eine Translationskraft auf die Mehlteigprobe, welche infolge der Krafterhaltung in gleicher Weise auf die Teigaufnahmeeinrichtung und das Kraftbeaufschlagungsmittel und mithin von dem Lastsensorsystem detektiert wird.

Gemäß einer modifizierten Ausführungsform ist vorgesehen, dass das Teigprobenmesssystem eine Rahmenanordnung aufweist, an der die Teigaufnahmeeinrichtung, das Lastsensorsystem und/oder das Kraftbeaufschlagungsmittel mittelbar oder unmittelbar angeordnet ist oder sind. Die Teigaufnahmeeinrichtung ist im Sinne einer mittelbaren Anordnung zumindest derart nicht fix mit der Rahmenanordnung verbunden, da sie abhängig von der Hubposition der Hubeinrichtung höhenverstellbar ist. Das Kraftbeaufschlagungsmittel kann beispielhaft über das Lastsensorsystem an der Rahmenanordnung angeordnet sein. Die Rahmenanordnung kann eine Blechanordnung, eine Fachwerkkonstruktion oder eine Kombination daraus sein. Möglich sind auch andere Ausgestaltungen.

Gemäß einer modifizierten Ausführungsform ist vorgesehen, dass die Hubeinrichtung mit der Rahmenanordnung verbunden ist, wobei die Teigaufnahmeeinrichtung über die Hubeinrichtung an der Rahmenanordnung angeordnet ist. Die Nutzung einer Hubeinrichtung erlaubt eine präzise Höhenverstellung der Teigaufnahmeeinrichtung. Um den Hub von der Hubeinrichtung auf die Teigaufnahmeeinrichtung zu übertragen, ist die Teigaufnahmeeinrichtung mit Vorzug mit einem Deckel der Hubeinrichtung verbunden.

Gemäß einer modifizierten Ausführungsform ist vorgesehen, dass
das Lastsensorsystem mit der Rahmenanordnung verbunden ist, und wobei
das Kraftbeaufschlagungsmittel über das Lastsensorsystem an der Rahmenanordnung angeordnet ist. Eine solche Konfiguration erlaubt weniger störanfällige Messungen gegenüber konventionellen Teigprobenmesssystemen.

Gemäß einer modifizierten Ausführungsform ist vorgesehen, dass
das Lastsensorsystem und das Kraftbeaufschlagungsmittel derart ausgebildet und angeordnet sind, dass die Kraftbeaufschlagungshauptachse und die Lastdetektionshauptachse parallel, vorzugsweise koaxial, zueinander verlaufen. Eine parallele Achsführung ermöglicht einen gleichbleibenden Kraftfluss zwischen den beteiligten Komponenten. Wären diese windschief angeordnet, würde dies mit entsprechenden Anteilen in die Messergebnisse einfließen. Hiervon unabhängig bevorzugt, jedoch in Kombination besonders bevorzugt, ist das Kraftbeaufschlagungsmittel im Wesentlichen unflexibel. Die Unflexiblität hat den Vorteil, dass die Parallelität, insbesondere die Koaxialität, der Achsen auch unter einen gewissen Last bestehen bleibt und sich nicht durch Materialverformung verändert. Im Wesentlichen unflexibel bedeutet hierbei für den Fachmann, dass ein Minimalmaß an Flexibilität grundsätzlich zulässig ist, um einen ungewollten Sprödbruch des Kraftbeaufschlagungsmittels zu verhindern. Das Kraftbeaufschlagungsmittel kann beispielsweise ausgebildet sein aus Metall oder aus Hartplastik. Als Weichplastik werden besonders biegsame Kunststoffe bezeichnet. Dies sind vor allem Weich-PVC und Polyolefine, insbesondere Polyethylen. Steifere Kunststoffe werden demgegenüber Hartplastik genannt, während man bei Elastomeren von (synthetischem) Gummi spricht. Als Hartplastik bezeichnete Kunststoffe können sowohl zur Gruppe der Thermoplaste wie der Duroplaste gehören, wobei es sich bei den meisten Duroplasten um Hartplastik handelt. Ein metallisches Kraftbeaufschlagungsmittel kann insbesondere aus Stahl bestehen.

Gemäß einer modifizierten Ausführungsform ist vorgesehen, dass
das Kraftbeaufschlagungsmittel derart hakenförmig ausgebildet ist,
dass es einen oberen Hakenabschnitt aufweist, um die Teigprobe entlang der Kraftbeaufschlagungshauptachse infolge der translatorischen Bewegung der Teigaufnahmeeinrichtung mit der Translationskraft zu beaufschlagen, und
dass es einen unteren Hakenabschnitt aufweist, um die auf die Teigprobe wirkende Translationskraft entlang der Lastdetektionshauptachse in das Lastsensorsystem zu führen. Grundsätzlich kann ein einziger Haken verwendet werden oder es können mehrere Haken seitlich aufeinander folgend zu einem Kraftbeaufschlagungsmittel aus mehreren Einzelhakensegmenten zusammengefügt werden. Ein solches Kraftbeaufschlagungsmittel hat den Vorteil, dass es schnell einsatzbereit ist zum Vermessen der Teigprobe. Die Hakenform ermöglicht ein Widerlagern der Teigprobe derart, dass die Teigprobe einerseits sicher gelagert ist und andererseits möglichst wenig mit dem Kraftbeaufschlagungsmittel interagiert, sodass das Maß an Hygiene erhöht wird.

Gemäß einer modifizierten Ausführungsform ist vorgesehen, dass
der obere Hakenabschnitt eine horizontale Erstreckungskomponente aufweist, wobei der obere Hakenabschnitt entlang der horizontalen Erstreckungskomponente vorzugsweise eine Bogenform aufweist, um die Teigprobe im oberen Bereich der Bogenform entlang der Kraftbeaufschlagungshauptachse infolge der translatorischen Bewegung der Teigaufnahmeeinrichtung mit der Translationskraft zu beaufschlagen. Die Bogenform gewährleistet eine zuverlässig reproduzierbare Zugbelastung.

Gemäß einer modifizierten Ausführungsform ist vorgesehen, dass
der untere Hakenabschnitt eine horizontale Erstreckungskomponente aufweist. Bevorzugt weist der untere Hakenabschnitt entlang der horizontalen Erstreckungskomponente eine Schwungform auf. Die bevorzugte Schwungform kann sich insbesondere in Richtung der Kraftbeaufschlagungshauptachse erstrecken, um das Maß des wirkenden Moments zu reduzieren und damit Störgrößen zu beseitigen. Besonders bevorzugt ist die Schwungform als S-Form ausgebildet, um die auf die Teigprobe wirkende Translationskraft entlang der Lastdetektionshauptachse und entlang der Kraftbeaufschlagungshauptachse in das Lastsensorsystem zu führen. Dies stellt sicher, dass keine Momente entstehen, welche das Messergebnis negativ beeinflussen. Es sei darauf verwiesen, dass Momente stets ein Produkt aus Last und Strecke sind, sodass die Strecke möglichst gering sein sollte, um Störgrößen zu reduzieren.

Gemäß einer modifizierten Ausführungsform ist vorgesehen, dass die Hubeinrichtung ein Antriebssystem, eine Hubsäule und ein Rechensystem aufweist. Das Antriebssystem weist vorzugsweise ein Getriebe auf.

Die Hubsäule ist mittels des Antriebssystems mit einem Verbindungsmittel, vorzugsweise stufenlos, verstellbar.

Das Verbindungsmittel ist verbindend zwischen der Hubsäule und der Teigaufnahmeeinrichtung zu derer translatorischen Bewegung angeordnet. Vorzugsweise ist das Verbindungsmittel hierzu endseitig an der Hubsäule angeordnet.

Ferner umfasst das Teigprobenmesssystem bevorzugt ein Rechensystem, das ausgebildet ist, den Streckenbetrag des Hubs der Hubeinrichtung aus Betriebsdaten der Hubsäule herzuleiten. Die Betriebsdaten weisen insbesondere Strom zum Antrieb des Antriebsmotors und/oder eine Stellungsänderung des Getriebes auf.

Gemäß einer modifizierten Ausführungsform ist vorgesehen, dass
die Teigaufnahmeeinrichtung und/oder das Kraftbeaufschlagungsmittel lösbar am Teigprobenmesssystem angeordnet sind. Diese modulare Lösung ermöglicht die Verwendung unterschiedlicher Teigaufnahmeeinrichtungen sowie Kraftbeaufschlagungsmittel. Mithin kann dasselbe Teigprobenmesssystem nach einem Austausch der Teigaufnahmeeinrichtung und/oder des Kraftbeaufschlagungsmittels für Zug- sowie Druckversuche an Teigproben verwendet werden. Bevorzugt zusätzlich, jedoch grundsätzlich von obigen Merkmalen unabhängig, ist vorgesehen, dass das Kraftbeaufschlagungsmittel als Zughaken oder als Druckkörper ausgebildet ist. Mithin erweitert diese Merkmalskombination die Vielseitigkeit des Teigprobenmesssystems.

Vorteilhaft ist außerdem ein Verfahren zur Bestimmung von Eigenschaften einer Teigprobe mit einem Teigprobenmesssystem, welches zumindest teilweise vorstehende Merkmale der vorbeschriebenen modifizierten Ausführungsformen aufweist. Das Verfahren weist vorzugsweise mindestens folgende Schritte auf:
- Anordnen der Teigprobe in der Teigaufnahmeeinrichtung;
- Bewegen der Teigaufnahmeeinrichtung entlang der Teigaufnahmenlängsachse in Richtung des Kraftbeaufschlagungsmittels;
- Auftreffen der Teigprobe auf das Kraftbeaufschlagungsmittel;
- Weiterbewegen der Teigaufnahmeeinrichtung entlang der Teigaufnahmenlängsachse in gleichbleibender Richtung;
- Detektieren, mittels des Lastsensorsystems, der auf die Teigprobe wirkenden Translationskraft.

Beispielhaft kann mit dem Verfahren ein Teigling, eingelegt in einer Teigschale, auf seine Eigenschaften untersucht werden.

Hierfür wird die Teigschale, bestückt mit einem längs gewirkten Teigling, in den Träger eingelegt. Bei Start der Messung verfährt die Hubsäule konstant vertikal nach oben, der Teig kommt in Kontakt mit dem Zughaken. Der Zughaken ist direkt gekoppelt mit einem Lastsensor. Abhängig vom Verfahrweg stellt sich eine Dehnung im Teigling ein. Die gemessene Last wird konstant erfasst und über den Verfahrweg in einem Diagramm aufgetragen bis der Probenkörper reißt.

Das Anordnen der Teigprobe in der Teigaufnahmeeinrichtung ist vorgesehen, um die Teigprobe derart zur Untersuchung einzurichten, dass sie während der Bestimmung ihrer Eigenschaften nicht von der Teigaufnahmeeinrichtung herunterfällt. Dabei kann die Teigprobe beispielhaft auf die Teigaufnahmeeinrichtung aufgelegt oder alternativ eingespannt werden. Weitere Optionen sind An- oder Durchbohren mit Fixiermitteln sowie eine Kombination der vorstehenden Mittel sowie grundsätzlich weitere Fixiermöglichkeiten.

Das Bewegen der Teigaufnahmeeinrichtung entlang der Teigaufnahmenlängsachse in Richtung des Kraftbeaufschlagungsmittels erfolgt mit Vorteil mit gleichmäßiger Geschwindigkeit. Eine ungleichmäßige und zu starke Beschleunigung könnte zum Beispiel dazu führen, dass die Massenträgheit verfälschend in das Messergebnis einfließt, sodass dies bevorzugt zu vermeiden ist.

Als Auftreffen der Teigprobe auf das Kraftbeaufschlagungsmittel ist der Moment zu verstehen, sobald ein physischer Kontakt zwischen der Teigprobe und dem Kraftbeaufschlagungsmittel vorliegt. Sodann erfolgt ein Kraftaustausch zwischen der Teigprobe und dem Kraftbeaufschlagungsmittel, welcher vom Lastsensorsystem als Translationskraft detektiert wird.

Anschließend erfolgt das Weiterbewegen der Teigaufnahmeeinrichtung entlang der Teigaufnahmenlängsachse in gleichbleibender Richtung. Während dieses Prozessschritts werden die Messdaten infolge des Teigprobenverhaltens vom Translationskraft detektiert. Spätestens sobald die Teigprobe zerstört ist, beispielsweise bei einem Bruch während eines Druckversuchs oder bei einem Riss während eines Zugversuchs, endet der physische Kontakt zwischen der Teigprobe und dem Kraftbeaufschlagungsmittel, sodass das Lastsensorsystem keine weitere Translationskraft detektiert. Alternativ ist es auch möglich, dass das Weiterbewegen der Teigaufnahmeeinrichtung nach einer definierten Zeit oder einem definierten Teigprobenverhalten endet.

Ebenso erfolgt das Detektieren, mittels des Lastsensorsystems, der auf die Teigprobe wirkenden Translationskraft. Dieser Prozessschritt ist nicht unbedingt chronologisch zu verstehen als Schritt nach dem Weiterbewegen der Teigaufnahmeeinrichtung, wobei dies nicht ausgeschlossen ist. Bevorzugt erfolgt das Detektieren vor oder mit dem Auftreffen der Teigprobe auf das Kraftbeaufschlagungsmittel. So ist es auch möglich, dass das Lastsensorsystem permanent eingeschaltet ist und aus Gründen der erst dann möglichen Datenübertragung im Moment des Auftreffens der Teigprobe auf das Kraftbeaufschlagungsmittel mit der Detektion beginnt.

Gemäß einer modifizierten Maßnahme ist vorgesehen, dass
das Weiterbewegen der Teigaufnahmeeinrichtung entlang der Teigaufnahmenlängsachse in gleichbleibender Richtung erfolgt, bis ein Materialversagen der Teigprobe erfolgt, beispielsweise durch ein Reißen der Teigprobe. Dies ermöglicht eine möglichst vollständige Datenerfassung der Teigprobeneigenschaften vom Beginn des physischen Kontakts zwischen der Teigprobe und dem Kraftbeaufschlagungsmittel bis zu dessen Ende.

Gemäß einer modifizierten Maßnahme ist vorgesehen, dass
ein Rechensystem, insbesondere das vorgenannte Rechensystem, ein Kraft-Weg-Diagramm erzeugt. Die vom Rechensystem generierte Belastungs-Dehnungskurve gibt Aufschluss über die rheologischen Eigenschaften der Teigprobe.

Vorteilhaft ist außerdem ein Computerprogramm, umfassend Befehle, die bei der Ausführung des Computerprogramms durch einen Computer diesen veranlassen, Schritte des vorgenannten Verfahrens teilweise oder vollständig auszuführen. Unter Verwendung eines entsprechend angepassten Computerprogramms können nicht nur ziehende Bewegungen beziehungsweise Messungen, sondern auch drückende Bewegungen beziehungsweise Messungen durchgeführt werden.

Vorteilhaft ist außerdem ein Datenträgersignal, das das vorgenannte Computerprogramm überträgt.

Vorteilhaft ist außerdem ein computerlesbares Medium, umfassend Befehle, die bei der Ausführung durch einen Computer diesen veranlassen, Schritte des vorgenannten Verfahrens teilweise oder vollständig auszuführen.

### Kurze Beschreibung der Zeichnungen

Nachfolgend wird das Teigprobenmesssystem unter Bezugnahme auf die anliegenden Zeichnungen anhand bevorzugter Ausführungsbeispiele näher erläutert. Die Formulierung Figur ist in den Zeichnungen mit Fig. abgekürzt.

In den Zeichnungen zeigen
- Fig. 1: eine schematische, perspektivische Ansicht eines Teigprobenmesssystems gemäß Stand der Technik;
- Fig. 2: eine schematische, seitliche Ansicht eines Teigprobenmesssystems gemäß einem ersten bevorzugten Ausführungsbeispiel;
- Fig. 3: eine schematische, seitliche Ansicht eines Teigprobenmesssystems gemäß einem zweiten bevorzugten Ausführungsbeispiel;
- Fig. 4: eine schematische, perspektivische Ansicht des Teigprobenmesssystems gemäß Figur 3; und
- Fig. 5: eine schematische, seitliche Ansicht eines Teigprobenmesssystems gemäß einem dritten bevorzugten Ausführungsbeispiel.

### Detaillierte Beschreibung der Ausführungsbespiele

Die beschriebenen Ausführungsbeispiele sind lediglich Beispiele von Teigprobenmesssystemen, die im Rahmen der Ansprüche auf vielfältige Weise modifiziert und/oder ergänzt werden können. Jedes Merkmal, das für ein bestimmtes Ausführungsbeispiel eines Teigprobenmesssystems beschrieben wird, kann eigenständig oder in Kombination mit anderen Merkmalen in einem beliebigen anderen Ausführungsbeispiel eines Teigprobenmesssystems genutzt werden. Jedes Merkmal, das für ein Ausführungsbeispiel einer bestimmten Anspruchskategorie beschrieben wird, kann auch in entsprechender Weise in einem Ausführungsbeispiel einer anderen Anspruchskategorie eingesetzt werden.

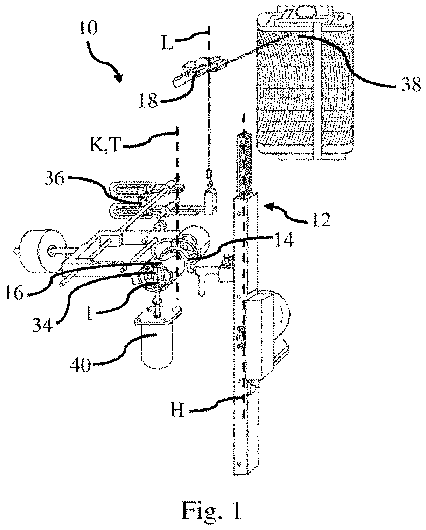

Figur 1 zeigt ein Teigprobenmesssystem 10 nach Stand der Technik. Dabei handelt es sich um ein Teigprobenmesssystem 10 zur Bestimmung von Eigenschaften einer Teigprobe. Das Teigprobenmesssystem 10 weist hierzu mindestens nachfolgende Komponenten auf.

Das vorbekannte Teigprobenmesssystem 10 weist eine Hubeinrichtung 12 auf, welche entlang einer Hubachse H in unterschiedliche Hubpositionen translatorisch höhenverstellbar ist. Die Höhenverstellbarkeit erfolgt derart, dass die Teigprobe abhängig von der Hubposition der Hubeinrichtung 12 infolge einer translatorischen Relativbewegung zwischen einem translatorisch bewegbaren Kraftbeaufschlagungsmittel 14 und einer ortsfesten Teigaufnahmeeinrichtung 16 mit einer Translationskraft beaufschlagbar ist. Weiterhin umfasst das Teigprobenmesssystem 10 ein translatorisch bewegbares Kraftbeaufschlagungsmittel 14, um die Teigprobe entlang einer Kraftbeaufschlagungshauptachse K infolge der translatorischen Bewegung des Kraftbeaufschlagungsmittels 14 entgegen der ortsfesten Teigaufnahmeeinrichtung 16 mit der Translationskraft zu beaufschlagen. Zudem weist das Teigprobenmesssystem 10 die entlang einer Teigaufnahmenlängsachse T ortsfeste Teigaufnahmeeinrichtung 16 auf, um die Teigprobe tragend widerzulagern. Dabei ist das Kraftbeaufschlagungsmittel 14 derart mit der Hubeinrichtung 12 verbunden, dass es bei einer Änderung der Hubposition der Hubeinrichtung 12 entlang der Hubachse H seinerseits entlang der Teigaufnahmenlängsachse T translatorisch bewegbar ausgebildet ist. Ebenso umfasst das Teigprobenmesssystem 10 ein Lastsensorsystem 18 zur Detektion der Translationskraft entlang einer Lastdetektionshauptachse L, wobei das Lastsensorsystem 18 zumindest mittelbar mit der Teigaufnahmeeinrichtung 16 verbunden ist. Die Teigaufnahmeeinrichtung 16 weist Fixiermittel 34 auf, die ausgebildet sind, die Teigprobe während einer Messung derart zu fixieren, dass das Messergebnis nicht verfälscht wird. Weiterhin weist das vorbekannte Teigprobenmesssystem 10 ein Hebelsystem 36 auf, um die Translationskraft von der Teigaufnahmeeinrichtung 16 auf das Lastsensorsystem 18 zu übertragen. Durch die erkennbare Hebelstrecke verstärken sich etwaige Messungenauigkeiten. Die vom Lastsensorsystem 18 detektierten Messdaten werden unmittelbar an einen Schreiber 38 übertragen, welcher beispielhaft ein Kraft-Weg-Diagramm erzeugt. Weiterhin weist das Teigprobenmesssystem 10 ein Dämpfungssystem 40 auf, um die Auswirkung von Fehleinflüssen, welche beispielhaft durch die Menge von Hebeln in den Messvorgang einwirken, zu reduzieren.

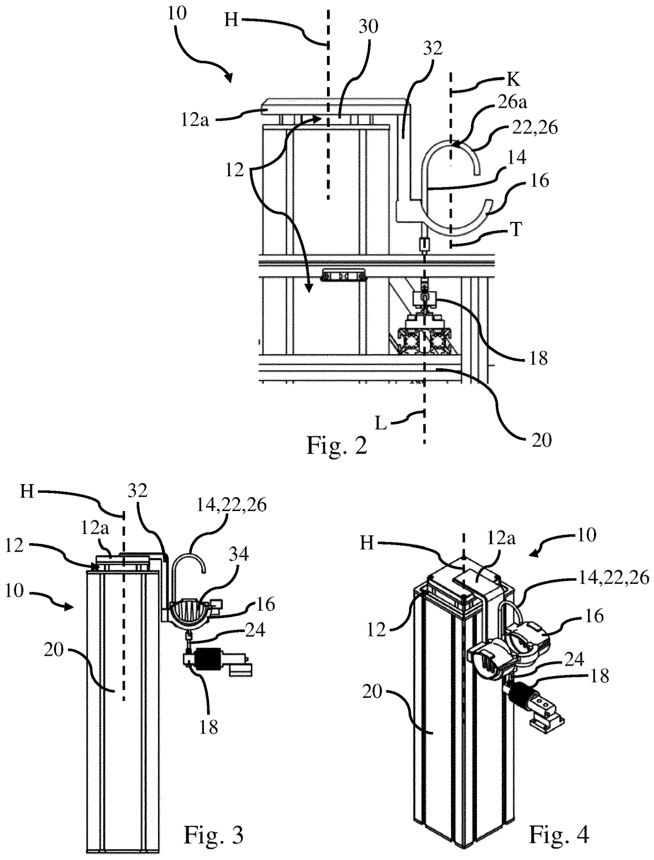

Die Figuren 2 bis 5 zeigen ein Teigprobenmesssystem 10 zur Bestimmung von Eigenschaften einer Teigprobe. Das Teigprobenmesssystem 10 weist hierzu mindestens nachfolgende Komponenten auf.

So weist das Teigprobenmesssystem 10 eine Hubeinrichtung 12 auf, welche entlang einer Hubachse H in unterschiedliche Hubpositionen translatorisch höhenverstellbar ist. Die Höhenverstellbarkeit erfolgt derart, dass die Teigprobe abhängig von der Hubposition der Hubeinrichtung 12 infolge einer translatorischen Relativbewegung zwischen einem ortsfesten Kraftbeaufschlagungsmittel 14 und einer translatorisch bewegbaren Teigaufnahmeeinrichtung 16 mit einer Translationskraft beaufschlagbar ist. Weiterhin umfasst das Teigprobenmesssystem 10 ein ortsfestes Kraftbeaufschlagungsmittel 14, um die Teigprobe entlang einer Kraftbeaufschlagungshauptachse K infolge der translatorischen Bewegung der Teigaufnahmeeinrichtung 16 mit der Translationskraft zu beaufschlagen. Zudem weist das Teigprobenmesssystem 10 eine entlang einer Teigaufnahmenlängsachse T translatorisch bewegbare Teigaufnahmeeinrichtung 16 auf, um die Teigprobe tragend widerzulagern. Dabei ist die Teigaufnahmeeinrichtung 16 derart mit der Hubeinrichtung 12 verbunden, dass die Teigaufnahmeeinrichtung 16 bei einer Änderung der Hubposition der Hubeinrichtung 12 entlang der Hubachse H ihrerseits entlang der Teigaufnahmenlängsachse T translatorisch bewegbar ausgebildet ist. Ebenso umfasst das Teigprobenmesssystem 10 ein Lastsensorsystem 18 zur Detektion der Translationskraft entlang einer Lastdetektionshauptachse L, wobei das Lastsensorsystem 18 zumindest mittelbar mit dem Kraftbeaufschlagungsmittel 14 verbunden ist.

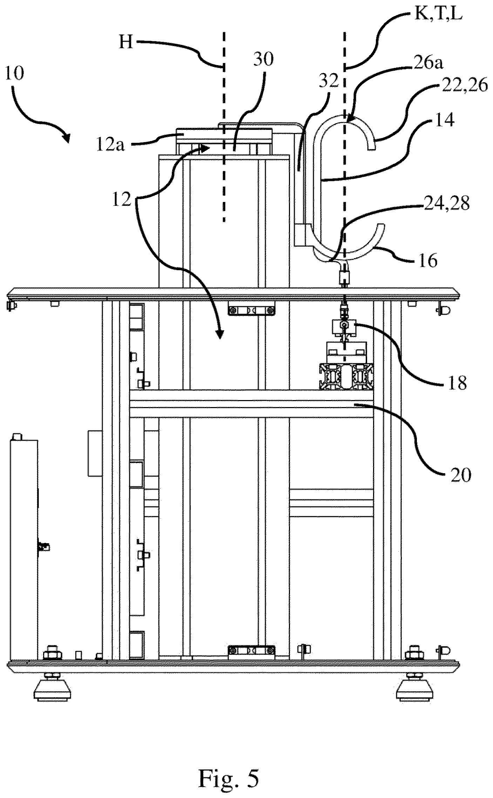

Den beispielhaften Ausführungsformen der Figuren 2 und 5 ist zu entnehmen, dass das Teigprobenmesssystem 10 eine Rahmenanordnung 20 aufweist. An dieser sind die Teigaufnahmeeinrichtung 16 über die Hubeinrichtung 12 und das Kraftbeaufschlagungsmittel 14 über das Lastsensorsystem 18 angeordnet. Die Hubeinrichtung 12 ist derart mit der Rahmenanordnung 20 verbunden, dass die Teigaufnahmeeinrichtung 16 über die Hubeinrichtung 12 an der Rahmenanordnung 20 angeordnet ist. Weiterhin ist das Lastsensorsystem 18 mit der Rahmenanordnung 20 verbunden, wobei das Kraftbeaufschlagungsmittel 14 über das Lastsensorsystem 18 an der Rahmenanordnung 20 angeordnet ist.

Gegenüber den Figuren 2 und 5 modifiziert ist den beispielhaften Ausführungsformen der Figuren 3 und 4 zu entnehmen, dass das Teigprobenmesssystem 10 ebenfalls eine Rahmenanordnung 20 aufweist. An dieser ist die Teigaufnahmeeinrichtung 16 über die Hubeinrichtung 12 angeordnet. Die Hubeinrichtung 12 ist derart mit der Rahmenanordnung 20 verbunden, sodass die Teigaufnahmeeinrichtung 16 über die Hubeinrichtung 12 an der Rahmenanordnung 20 angeordnet ist. Das Kraftbeaufschlagungsmittel 14 ist mit dem Lastsensorsystem 18 verbunden, welches beispielhaft auf einer ortsfesten Unterlage, zum Beispiel auf einem Tisch, angeordnet sein kann. Die Befestigung des Lastsensorsystems 18 ist nicht näher dargestellt.

Um den Hub von der Hubeinrichtung 12 auf die Teigaufnahmeeinrichtung 16 zu übertragen, ist die Teigaufnahmeeinrichtung 16 gemäß den Figuren 2 bis 5 mit Vorzug mit einem Deckel 12a der Hubeinrichtung 12 verbunden. Die Verbindung der Teigaufnahmeeinrichtung 16 mit dem Deckel 12a der Hubeinrichtung 12 kann insbesondere mittelbar oder unmittelbar erfolgen. Dies ermöglicht eine kompakte Bauweise des Teigprobenmesssystems 10, da zusätzliche, platzeinnehmende Komponenten eingespart werden können. Dieses Merkmal kann mit weiteren Merkmalen der Ausführungsformen kombiniert werden oder isoliert von diesen genutzt werden.

Das Lastsensorsystem 18 und das Kraftbeaufschlagungsmittel 14 sind derart ausgebildet und angeordnet, dass die Kraftbeaufschlagungshauptachse K und die Lastdetektionshauptachse L gemäß den Ausführungsformen nach den Figuren 3, 4 und 5 koaxial und gemäß der Ausführungsform nach Figur 2 parallel zueinander verlaufen.

Wie beispielhaft in Figur 5 zu sehen ist, ist das Kraftbeaufschlagungsmittel 14 derart hakenförmig ausgebildet, dass es einen oberen Hakenabschnitt 22 aufweist, um die Teigprobe entlang der Kraftbeaufschlagungshauptachse K infolge der translatorischen Bewegung der Teigaufnahmeeinrichtung 16 mit der Translationskraft zu beaufschlagen. Weiterhin weist das Kraftbeaufschlagungsmittel 14 einen unteren Hakenabschnitt 24 auf, um die während einer Messung auf die Teigprobe wirkende Translationskraft entlang der Lastdetektionshauptachse L in das Lastsensorsystem 18 zu führen.

Mit Vorzug weist der obere Hakenabschnitt 22 eine horizontale Erstreckungskomponente auf, wobei der obere Hakenabschnitt 22 entlang der horizontalen Erstreckungskomponente insbesondere eine Bogenform 26 aufweist. Dies ermöglicht ein Beaufschlagen der Teigprobe im oberen Bereich 26a der Bogenform 26 entlang der Kraftbeaufschlagungshauptachse K infolge der translatorischen Bewegung der Teigaufnahmeeinrichtung 16 mit der Translationskraft.

Weiter ist mit Vorzug vorgesehen, dass der untere Hakenabschnitt 24 eine horizontale Erstreckungskomponente aufweist. Dieser untere Hakenabschnitt 24 weist entlang der horizontalen Erstreckungskomponente bevorzugt eine Schwungform 28 auf. Diese Schwungform 28 ist besonders bevorzugt als S-Form ausgebildet, um die auf die Teigprobe wirkende Translationskraft entlang der Lastdetektionshauptachse L und entlang der Kraftbeaufschlagungshauptachse K in das Lastsensorsystem 18 zu führen.

Die Figuren 2 bis 5 zeigen das Teigprobenmesssystem mit einer jeweiligen Hubeinrichtung 12. Diese weist auf ein nicht genauer gezeigtes Antriebssystem und eine mittels des Antriebssystems verstellbare Hubsäule 30 mit einem Verbindungsmittel 32. Das Antriebssystem weist vorzugsweise ein Getriebe auf, welches nicht näher dargestellt ist. Die Hubsäule 30 ist vorzugsweise stufenlos verstellbar. Dabei ist das Verbindungsmittel 32 verbindend zwischen der Hubsäule 30 und der Teigaufnahmeeinrichtung 12 zu dessen translatorischen Bewegung angeordnet. Hierzu ist das Verbindungsmittel 32 vorzugsweise endseitig an der Hubsäule 30 angeordnet. Ferner weist das Teigprobenmesssystem 10 bevorzugt ein nicht näher dargestelltes Rechensystem auf. Dieses ist ausgebildet, um den Streckenbetrag des Hubs der Hubeinrichtung 12 aus Betriebsdaten der Hubsäule 22 herzuleiten. Passende Betriebsdaten sind insbesondere Strom zum Antrieb des Antriebsmotors und/oder eine Stellungsänderung des Getriebes.

Nicht im Detail dargestellt, jedoch grundsätzlich bevorzugt, ist vorgesehen, dass die Teigaufnahmeeinrichtung 12 und/oder das Kraftbeaufschlagungsmittel 14 lösbar am Teigprobenmesssystem 10 angeordnet sind. Unabhängig von den anderen Merkmalen kann dies beispielhaft als Schraub-, Magnet und/oder Bajonettverschluss erfolgen. Andere Lösungen sind ebenfalls möglich.

Weiterhin ist in den Figuren 2 bis 5 gezeigt, dass das Kraftbeaufschlagungsmittel 14 als Zughaken ausgebildet ist. Optional ist auch möglich, dass das

Kraftbeaufschlagungsmittel 14 als Druckkörper ausgebildet ist. Wenn das Kraftbeaufschlagungsmittel 14 lösbar, und damit modular, ausgebildet ist, kann das Teigprobenmesssystem 10 für unterschiedliche Anwendungen genutzt werden. Optional, und in Kombination oder unabhängig von weiteren Merkmalen, kann das Teigprobenmesssystem 10 mit einem entsprechenden Druckkörper als Kraftbeaufschlagungsmittel 14 ausschließlich für Druckmessungen verwendet werden.

Zur Bestimmung von Eigenschaften der Teigprobe mit dem Teigprobenmesssystem 10 werden mindestens folgende Verfahrensschritte ausgeführt. Zunächst wird die Teigprobe in der Teigaufnahmeeinrichtung 16 angeordnet. Dies kann beispielhaft dadurch erfolgen, dass die Teigprobe mit Fixiermitteln 34 durchstochen wird, sodass sie infolge geringer Krafteinflüsse nicht ungewollt von der Teigaufnahmeeinrichtung 16 herunterfallen kann. Anschließend wird die Teigaufnahmeeinrichtung 16 entlang der Teigaufnahmenlängsachse T in Richtung des Kraftbeaufschlagungsmittels 14 nach oben bewegt. Während der Annäherung trifft die Teigprobe auf das Kraftbeaufschlagungsmittel 14 auf. Ab diesem Moment kann das Lastsensorsystem 18 eine vertikal wirkende Last detektieren, welche zusammengefasst als Translationskraft benannt ist, wobei die physikalischen Wechselwirkungsgesetze angewandt werden. Nach dem Auftreffen zwischen der Teigprobe und dem Kraftbeaufschlagungsmittel 14 erfolgt, insbesondere gleichmäßig, also ohne Verzögerung, ein Weiterbewegen der Teigaufnahmeeinrichtung 16 entlang der Teigaufnahmenlängsachse T in gleichbleibender Richtung. Dieses Weiterbewegen der Teigaufnahmeeinrichtung 16 entlang der Teigaufnahmenlängsachse T in gleichbleibender Richtung bewirkt eine Dehnung der Teigprobe und erfolgt bis ein Materialversagen der Teigprobe erfolgt, beispielsweise durch ein Reißen der Teigprobe.

Das nicht dargestellte Rechensystem erzeugt ein Kraft-Weg-Diagramm, welches Informationen über die Eigenschaften einer Teigprobe bietet.

Nicht dargestellt, jedoch im Rahmen der Erfindung einzeln oder gemeinsam vorgesehen sind ein Computerprogramm, ein Datenträgersignal und ein computerlesbares Medium.

### Bezugszeichenliste

- 1: Teigprobe
- 10: Teigprobenmesssystem
- 12: Hubeinrichtung
- 12a: Deckel der Hubeinrichtung
- 14: Kraftbeaufschlagungsmittel
- 16: Teigaufnahmeeinrichtung
- 18: Lastsensorsystem
- 20: Rahmenanordnung
- 22: Oberer Hakenabschnitt des Kraftbeaufschlagungsmittels
- 24: Unterer Hakenabschnitt des Kraftbeaufschlagungsmittels
- 26: Bogenform des oberen Hakenabschnitts
- 26a: Oberer Bereich der Bogenform des oberen Hakenabschnitts
- 28: Schwungform des unteren Hakenabschnitts
- 30: Hubsäule der Hubeinrichtung
- 32: Verbindungsmittel
- 34: Fixiermittel
- 36: Hebelsystem
- 38: Schreiber
- 40: Dämpfungssystem

- H: Hubachse der Hubeinrichtung
- K: Kraftbeaufschlagungshauptachse des Kraftbeaufschlagungsmittels
- T: Teigaufnahmenlängsachse der Teigaufnahmeeinrichtung
- L: Lastdetektionshauptachse des Lastsensorsystems

## Patentansprüche

1. Teigprobenmesssystem zur Bestimmung von Eigenschaften einer Teigprobe, wobei das Teigprobenmesssystem (10) mindestens aufweist:
- eine Hubeinrichtung (12), welche entlang einer Hubachse (H) derart in unterschiedliche Hubpositionen translatorisch höhenverstellbar ist, dass die Teigprobe abhängig von der Hubposition der Hubeinrichtung (12) infolge einer translatorischen Relativbewegung zwischen einem ortsfesten Kraftbeaufschlagungsmittel (14) und einer translatorisch bewegbaren Teigaufnahmeeinrichtung (16) mit einer Translationskraft beaufschlagbar ist;
- ein ortsfestes Kraftbeaufschlagungsmittel (14), um die Teigprobe entlang einer Kraftbeaufschlagungshauptachse (K) infolge der translatorischen Bewegung der Teigaufnahmeeinrichtung (16) mit der Translationskraft zu beaufschlagen;
- eine entlang einer Teigaufnahmenlängsachse (T) translatorisch bewegbare Teigaufnahmeeinrichtung (16), um die Teigprobe tragend widerzulagern,
wobei die Teigaufnahmeeinrichtung (16) derart mit der Hubeinrichtung (12) verbunden ist, dass die Teigaufnahmeeinrichtung (16) bei einer Änderung der Hubposition der Hubeinrichtung (12) entlang der Hubachse (H) ihrerseits entlang der Teigaufnahmenlängsachse (T) translatorisch bewegbar ausgebildet ist; und
- ein Lastsensorsystem (18) zur Detektion der Translationskraft entlang einer Lastdetektionshauptachse (L), wobei das Lastsensorsystem (18) zumindest mittelbar mit dem Kraftbeaufschlagungsmittel (14) verbunden ist.

2. Teigprobenmesssystem nach Anspruch 1, wobei
das Teigprobenmesssystem (10) eine Rahmenanordnung (20) aufweist, an der die Teigaufnahmeeinrichtung (16), das Lastsensorsystem (18) und/oder das Kraftbeaufschlagungsmittel (14) mittelbar oder unmittelbar angeordnet ist oder sind.

3. Teigprobenmesssystem nach Anspruch 2, wobei
die Hubeinrichtung (12) mit der Rahmenanordnung (20) verbunden ist, und wobei die Teigaufnahmeeinrichtung (16) über die Hubeinrichtung (12) an der Rahmenanordnung (20) angeordnet ist.

4. Teigprobenmesssystem nach Anspruch 2 oder 3, wobei
das Lastsensorsystem (18) mit der Rahmenanordnung (20) verbunden ist, und wobei das Kraftbeaufschlagungsmittel (14) über das Lastsensorsystem (18) an der Rahmenanordnung (20) angeordnet ist.

5. Teigprobenmesssystem nach mindestens einem der vorgenannten Ansprüche, wobei
das Lastsensorsystem (18) und das Kraftbeaufschlagungsmittel (14) derart ausgebildet und angeordnet sind, dass die Kraftbeaufschlagungshauptachse (K) und die Lastdetektionshauptachse (L) parallel, vorzugsweise koaxial, zueinander verlaufen.

6. Teigprobenmesssystem nach Anspruch 5, wobei
das Kraftbeaufschlagungsmittel (14) derart hakenförmig ausgebildet ist,
dass es einen oberen Hakenabschnitt (22) aufweist, um die Teigprobe entlang der Kraftbeaufschlagungshauptachse (K) infolge der translatorischen Bewegung der Teigaufnahmeeinrichtung (16) mit der Translationskraft zu beaufschlagen, und
dass es einen unteren Hakenabschnitt (24) aufweist, um die auf die Teigprobe wirkende Translationskraft entlang der Lastdetektionshauptachse (L) in das Lastsensorsystem (18) zu führen.

7. Teigprobenmesssystem nach Anspruch 6, wobei
der obere Hakenabschnitt (22) eine horizontale Erstreckungskomponente aufweist, wobei der obere Hakenabschnitt (22) entlang der horizontalen Erstreckungskomponente vorzugsweise eine Bogenform (26) aufweist, um die Teigprobe im oberen Bereich (26a) der Bogenform (26) entlang der Kraftbeaufschlagungshauptachse (K) infolge der translatorischen Bewegung der Teigaufnahmeeinrichtung (16) mit der Translationskraft zu beaufschlagen.

8. Teigprobenmesssystem nach Anspruch 6 oder 7, wobei
der untere Hakenabschnitt (24) eine horizontale Erstreckungskomponente aufweist, wobei der untere Hakenabschnitt (24) entlang der horizontalen Erstreckungskomponente bevorzugt eine Schwungform (28) aufweist, besonders bevorzugt eine als S-Form ausgebildete Schwungform (28), um die auf die Teigprobe wirkende Translationskraft entlang der Lastdetektionshauptachse (L) und entlang der Kraftbeaufschlagungshauptachse (K) in das Lastsensorsystem (18) zu führen.

9. Teigprobenmesssystem nach mindestens einem der vorgenannten Ansprüche, wobei
die Hubeinrichtung (12) aufweist:
- ein Antriebssystem, vorzugsweise mit einem Getriebe, und
- eine mittels des Antriebssystems, vorzugsweise stufenlos, verstellbare Hubsäule (30) mit einem Verbindungsmittel (32);
wobei das Verbindungsmittel (32) verbindend zwischen der Hubsäule (30) und der Teigaufnahmeeinrichtung (12) zu derer translatorischen Bewegung angeordnet ist, wobei das Verbindungsmittel (32) vorzugsweise endseitig an der Hubsäule (30) angeordnet ist; wobei das Teigprobenmesssystem (10) bevorzugt ein Rechensystem aufweist, das ausgebildet ist, den Streckenbetrag des Hubs der Hubeinrichtung (12) aus Betriebsdaten der Hubsäule (22) herzuleiten,
wobei die Betriebsdaten insbesondere Strom zum Antrieb des Antriebsmotors und/oder eine Stellungsänderung des Getriebes aufweisen.

10. Teigprobenmesssystem nach mindestens einem der vorgenannten Ansprüche, wobei
die Teigaufnahmeeinrichtung (12) und/oder das Kraftbeaufschlagungsmittel (14) lösbar am Teigprobenmesssystem (10) angeordnet sind; und/oder wobei
das Kraftbeaufschlagungsmittel (14) als Zughaken oder als Druckkörper ausgebildet ist.

11. Verfahren zur Bestimmung von Eigenschaften einer Teigprobe mit einem Teigprobenmesssystem (10) nach einem der Ansprüche 1 bis 10, das Verfahren aufweisend mindestens folgende Schritte:
- Anordnen der Teigprobe in der Teigaufnahmeeinrichtung (16);
- Bewegen der Teigaufnahmeeinrichtung (16) entlang der Teigaufnahmenlängsachse (T) in Richtung des Kraftbeaufschlagungsmittels (14);
- Auftreffen der Teigprobe auf das Kraftbeaufschlagungsmittel (14);
- Weiterbewegen der Teigaufnahmeeinrichtung (16) entlang der Teigaufnahmenlängsachse (T) in gleichbleibender Richtung;
- Detektieren, mittels des Lastsensorsystems (18), der auf die Teigprobe wirkenden Translationskraft.

12. Verfahren zur Bestimmung von Eigenschaften einer Teigprobe nach Anspruch 11, wobei
das Weiterbewegen der Teigaufnahmeeinrichtung (16) entlang der Teigaufnahmenlängsachse (T) in gleichbleibender Richtung erfolgt bis ein Materialversagen der Teigprobe erfolgt, beispielsweise durch ein Reißen der Teigprobe.

13. Computerprogramm, umfassend Befehle, die bei der Ausführung des Computerprogramms durch einen Computer diesen veranlassen, ein Verfahren nach mindestens einem der vorgenannten Ansprüche 11 oder 12 auszuführen.

14. Datenträgersignal, das das Computerprogramm nach dem Anspruch 13 überträgt.

15. Computerlesbares Medium, umfassend Befehle, die bei der Ausführung durch einen Computer diesen veranlassen, ein Verfahren nach mindestens einem der Ansprüche 11 oder 12 auszuführen.
